# FASCICULE DE BREVET EUROPEEN

(11) **EP 0 610 467 B1**
(45) Date de publication et mention de la délivrance du brevet: **03.02.1999**
(21) Numéro de dépôt: 93916017.2
(22) Date de dépôt: 20.07.1993
(51) Int. Cl.: A61F 6/04

(54) **PRESERVATIF EQUIPE D'UNE PASTILLE RIGIDE DETROMPEUSE ET DE PROTECTION**
KONDOM MIT GEBRAUCHSSICHEREM SCHUTZ- UND ORIENTIERUNGSHILFSMITTEL
CONTRACEPTIVE SHEATH WITH ORIENTATION AND PROTECTION CAP

(30) Priorité: 28.08.1992 FR 9210353
(43) Date de publication de la demande: 17.08.1994
(73) Titulaire: BABLED, Raymond, F-95210 Sain-Gratien (FR)
(72) Inventeur: BABLED, Raymond, F-95210 Sain-Gratien (FR)
(74) Mandataire: Sauvage, Renée
(86) Numéro de dépôt international: FR9300737
(87) Numéro de publication internationale: WO9405237

(56) Documents cités:
- WO-A-90/12554
- DE-A- 3 902 093
- DE-U- 8 914 151
- US-A- 2 904 041

## Description

La présente invention concerne la combinaison d'un préservatif avec une pastille détrompeuse rigide pour préservatif, dont le pourtour est destiné à être engagé sous le bourrelet d'enroulement du préservatif, ladite pastille étant disposée sur l'extrémité et du côté extérieur de celui-ci.

Le document US-A-2 904 041 divulgue une pastille formant couvercle disposée du côté intérieur du préservatif, c'est-à-dire , du côté du préservatif destine à être en contact avec l'organe de l'utilisateur.

Un préservatif équipé d'une pastille disposée de son côté extérieur a été décrit dans la demande de brevet français BABLED n° 90-05994 du 14 mai 1990. Il s'agit essentiellement d'une pastille plate. Son but est d'éviter que l'utilisateur ne pose par mégarde le préservatif à l'envers. Ce but d'une telle pastille est même d'éviter que cet utilisateur ne transmette des germes pathogènes à sa partenaire en posant le préservatif dans le bon sens après une première tentative lors de laquelle, le présentant du mauvais côté, il aura amené en contact avec le gland du pénis la face extérieure de l'extrémité du préservatif.

Ainsi, une telle pastille possède un double rôle : il s'agit d'une pastille détrompeuse et de protection.

Une autre pastille de ce type est divulguée dans DE-A-39 02 093.

Le but de la présente invention est de perfectionner une telle pastille, en particulier en rendant plus sûr son maintien dans le bourrelet du préservatif jusqu' au moment précédant immédiatement la mise en place de ce dernier, ceci tout en permettant une éjection très facile de la pastille, ne nécessitant aucune intervention de l'utilisateur, cette éjection se produisant automatiquement dès le début de la pose.

Un autre but de l'invention est de proposer une pastille ayant une fonction détrompeuse et de protection améliorée.

A cet effet ,la présente invention concerne le dispositif selon la revendication 1, qui comprend, notamment, une pastille rigide de protection pour préservatif, ayant sur son pourtour une collerette plate destinée à être engagée sous le bourrelet d'enroulement du préservatif, ladite pastille étant disposée sur l'extrémité et du côté extérieur de celui-ci, ladite collerette entourant une partie de la pastille qui, vers l'extérieur, présente une surépaisseur par rapport au plan de ladite collerette.

Cette surépaisseur contribue, avec ladite collerette, au centrage et au maintien de la pastille dans le bourrelet du préservatif. Cette surépaisseur se présentant vers l'extérieur par rapport au plan de la collerette, permettra aussi à l'utilisateur de se rendre compte immédiatement au toucher, même dans l'obscurité, s'il tient le préservatif dans le sens correct ou s'il doit le retourner avant de le mettre en place.

La surépaisseur de la pastille est constituée d'une jupe d'appui latéral pour ledit bourrelet, cette jupe pouvant avoir une hauteur de par exemple 4 à 5 mm, combinée à un fond convexe vers l'extérieur, c est-à-dire une sorte de dôme sphérique ou de forme analogue, dont la base se raccorde à la collerette. Le fond, se raccordant, par sa périphérie, à la base de la jupe, peut avoir sensiblement la même hauteur que cette dernière, ou une hauteur légèrement supérieure, lui permettant d'apparaître légèrement en saillie au-dessus du bord de la jupe, et de contribuer ainsi à la reconnaissance au toucher, par l'utilisateur, du sens de pose correct.

Quant à la collerette, c'est-à-dire la partie plate de la pastille qui s'étend autour de ladite surépaisseur, elle aura toute largeur et toute forme lui permettant de s'engager suffisamment sous le bourrelet du préservatif pour que la pastille ne risque pas de s'en échapper fortuitement dans l'emballage, ou prématurément avant l'instant précédant immédiatement la mise en place sur le pénis. A cet effet, la largeur de la collerette pourra être de l'ordre de 1,2 à 2,5 mm environ, ces valeurs étant données à titre indicatif.

Si, vue en plan, la pastille a une forme circulaire, comme ce sera le cas le plus courant, la jupe aura aussi une forme circulaire, de sorte que la collerette, elle aussi circulaire, aura une largeur constante. Cette collerette peut être continue ou interrompue. Dans ce cas, elle peut être constituée par exemple par une série d'ergots, c'est-à-dire de petites saillies radiales, également espacées à la périphérie de la jupe.

Un mode d'exécution de l'invention va maintenant être décrit à titre d'exemple nullement limitatif, avec référence aux figures du dessin ci-annexé dans lequel :
- la figure 1 est une vue en coupe axiale d'un préservatif enroulé équipé d'une pastille rigide conforme à l'invention;
- la moitié supérieure de la figure 2 montre la forme de la pastille seule, vue en plan, la partie inférieure de cette figure montrant une variante pour la forme de la collerette; et
- la figure 3 est une vue en perspective de la pastille, à échelle plus réduite.

Sur les différentes figures, la colierette de la pastille rigide détrompeuse et de protection a été référencée 1, la jupe d'appui latéral 2, et le fond convexe 3. La pastille étant dans cet exemple de forme circulaire, on comprend que la jupe 2 est cylindrique, le fond convexe 3, ou dôme, étant sphérique ou de forme voisine. On voit que la pastille est maintenue avec sécurité dans le bourrelet B du préservatif P, en s'appuyant sous ce bourrelet par sa collerette 1 et latéralement contre ce bourrelet par sa jupe 2. Dans l'exemple on a supposé que le préservatif P possédait un réservoir R. On voit que ce réservoir se loge facilement à l'intérieur du dôme 3.

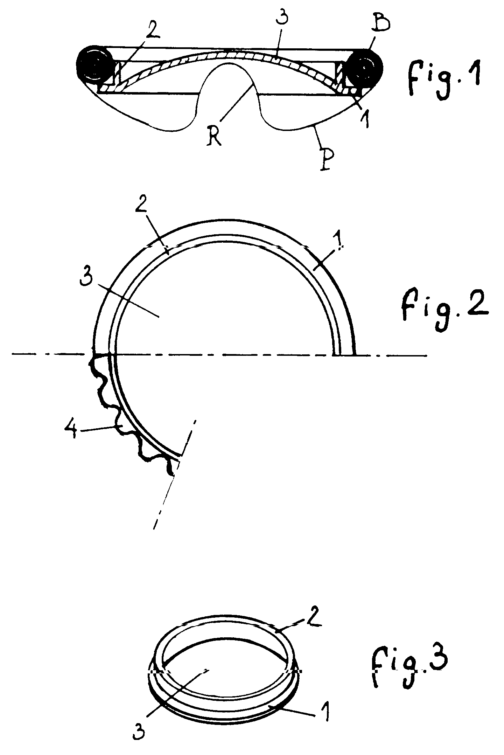

La partie supérieure de la figure 2 montre une collerette 1 circulaire et de largeur constante. A la partie inférieure de cette figure, on a montré la possibilité de lui donner la forme d'une série d'ergots 4, régulièrement répartis autour de la jupe 2.

Les diamètres extérieurs de la collerette 1 et de la jupe 2 sont bien entendu déterminés en prenant en compte le diamètre intérieur du bourrelet B d'un préservatif de dimensions normales, pour que le diamètre extérieur de ladite jupe soit légèrement supérieur au diamètre intérieur du bourrelet; de la sorte, le préservatif sera un peu distendu radialement par l'opération de pose de la pastille, ce qui assurera un excellent maintien de cette dernière. Ainsi, un diamètre extérieur courant pour la jupe 2 pourra être d'environ 28 mm, avec un diamètre de collerette 1 de l'ordre de 31 à 32mm, ces valeurs étant là encore données à titre purement indicatif.

La jupe 2 pourra avoir une hauteur approximative de 4 mm, et le fond convexe 3 une hauteur de l'ordre de 5mm.

Le fond convexe 3, en principe sphérique, aura un rayon de courbure adapté à celui de la jupe 2. Pour une jupe de diamètre extérieur compris par exemple entre 26 et 30 mm, et donc un diamètre de collerette 1 compris entre environ 30 et 35 mm, le rayon de courbure du fond 3 pourra être de l'ordre de 20 à 25 mm environ.

L'intérêt d'un tel fond 3 sphérique est, comme indiqué plus haut, de faciliter à l'utilisateur la reconnaissance du sens dans lequel il doit présenter le préservatif avant de le mettre sur son sexe; il va de soi qu'il le posera pour que la partie concave du fond de la pastille vienne coiffer le gland. Ce geste deviendra rapidement si naturel qu'il s'effectuera par réflexe, sans nécessiter une attention particulière. On comprend aussi que l'éjection de la pastille se produira aussi naturellement, sans nécessiter aucune intervention particulière, le préservatif étant posé de la façon habituelle, en faisant descendre le bourrelet B le long du pénis. Des le début du déroulement, la pastille, ayant joué son rôle détrompeur et de protection, sera automatiquement éjectée.

La pastille de l'invention présente encore l'avantage d'être facile à poser par le fabricant, dans une production en grandes séries. En raidissant les préservatifs, les pastilles faciliteront aussi leur conditionnement, ainsi que leur tenue dans les emballages, qu'ils soient constitués de pochettes carrées et scellées, ou sous tout autre forme.

La matière constituant les pastilles sera de préférence du polyéthylène alimentaire, ou tout autre matière synthétique appropriée.

## Revendications

1. Dispositif contraceptif et de protection contre les maladies sexuellement transmissibles comprenant, en combinaison :
a) un préservatif enroulé (P) présentant un bourrelet d' enroulement(B), et
b) une pastille rigide détrompeuse et de protection positionnée sous le bourrelet d'enroulement (B) et du côté extérieur du préservatif, côté qui est opposé au côté destiné à être en contact avec l'organe de l'utilisateur, ladite pastille comprenant:
une jupe d'appui latéral (2) comprenant une surface périphérique destinée à servir d'appui au bourrelet (B) dudit préservatif enroulé (P),
une collerette plate (1) qui s'étend radialement vers l'extérieur depuis la base de ladite jupe (2) et dont le pourtour est engagé sous le bourrelet d'enroulement (B) du préservatif, et
un fond convexe (3) disposé à l'intérieur de ladite jupe (2) et relié à la base de ladite jupe (2).

2. Dispositif selon la revendication 1, caractérisé en ce que la hauteur de ladite jupe (2) est d'environ 4 à 5 mm et son diamètre extérieur est compris entre environ 26 et 30 mm.

3. Dispositif selon la revendication 1 ou 2, caractérisé en ce que la hauteur dudit fond convexe (3) est sensiblement égale ou légèrement supérieure à celle de ladite jupe (2).

4. Dispositif selon l'une quelconque des revendications 1 à 3, caractérisé en ce que la largeur de ladite collerette (1) est d'environ 1,2 à 2,5 mm.

5. Dispositif selon l'une quelconque des revendications 1 à 4, caractérisé en ce que ladite collerette (1) est circulaire et a un diamètre de l'ordre de 30 à 35 mm.

6. Dispositif selon l'une quelconque des revendications 1 à 4, caractérisé en ce que ladite collerette (1) présente une série d'ergots périphériques (4).

7. Dispositif selon l'une quelconque des revendications 1 à 6, caractérisé en ce que ledit fond convexe (3) a la forme d'un dôme sphérique qui a un rayon de courbure de l'ordre de 20 à 25 mm.

8. Dispositif selon l'une quelconque des revendications 1 à 7, caractérisé en ce que ladite pastille est en polyéthylène alimentaire.

## Claims

1. Contraceptive device also giving protection against sexually transmissible diseases comprising, in combination:
a) a rolled sheath (P) with a rolling bead (B) and
b) a rigid protective orientating disc positioned underneath the rolling bead (B) and on the outside of the sheath, i.e. on the opposite side from that intended to be in contact with the user's member, the said disc comprising :
a lateral bearing skirt (2) comprising a peripheral surface against which the bead (B) of the said rolled sheath (P) is to bear,
a flat collar (1) that extends radially outwards from the base of the said skirt (2), its perimeter being engaged underneath the rolling bead (B) of the sheath, and
a convex hood (3) situated inside the said skirt (2) and joined to the base of the said skirt (2).

2. Device according to Claim 1, characterized in that the height of the said skirt (2) is approximately 4 to 5 mm and its external diameter is between approximately 26 and 30 mm.

3. Device according to Claim 1 or 2, characterized in that the height of the said convex hood is approximately equal to or slightly greater than that of the said skirt (2).

4. Device according to any one of Claims 1 to 3, characterized in that the width of the said collar (1) is approximately 1.2 to 2.5 mm.

5. Device according to any one of Claims 1 to 4, characterized in that the said collar (1) is circular and has a diameter of about 30 to 35 mm.

6. Device according to any one of Claims 1 to 4, characterized in that the said collar (1) comprises a series of peripheral nubs (4).

7. Device according to any one of Claims 1 to 6, characterized in that the said convex hood (3) is in the shape of a spherical dome with a radius of curvature of about 20 to 25 mm.

8. Device according to any one of Claims 1 to 7, characterized in that the said disc is of food-grade polyethylene.

## Patentansprüche

1. Vorrichtung zur Empfängnisverhütung und zum Schutz gegen Geschlechtskrankheiten, die in Kombination folgendes aufweist:
a) ein aufgerolltes Präservativ (P) mit einem Aufrollwulst(B) und
b) ein starres Unverwechselbarkeits- und Schutzplättchen, das unter dem Aufrollwulst (B) auf der Außenseite des Präservativs positioniert ist, die der Seite entgegengesetzt ist, die dazu bestimmt ist, mit dem Organ des Benutzers in Berührung zu kommen,
wobei dieses Plättchen folgendes aufweist:
einen seitlichen Stützkragen (2), der eine Umfangsfläche aufweist, die dazu bestimmt ist, dem Wulst (B) des aufgerollten Präservativs (P) als Auflagefläche zu dienen,
einen flachen Flansch (1), der sich von der Basis des Kragens (2) aus radial nach außen erstreckt und dessen Umfang unter den Aufrollwulst (B) des Präservativs eingeführt ist, und
einen konvexen Boden (3), der innerhalb des Kragens (2) angeordnet ist und mit der Basis des Kragens (2) verbunden ist.

2. Vorrichtung nach Anspruch 1, dadurch gekennzeichnet, daß die Höhe des Kragens (2) etwa 4 bis 5 mm und sein Außendurchmesser etwa 26 bis 30 mm beträgt.

3. Vorrichtung nach Anspruch 1 oder 2, dadurch gekennzeichnet, daß die Höhe des konvexen Bodens (3) im wesentlichen gleich der Höhe des Kragens (2) oder etwas größer ist.

4. Vorrichtung nach einem der Ansprüche 1 bis 3, dadurch gekennzeichnet, daß die Breite des Flansches (1) etwa 1,2 bis 2,5 mm beträgt.

5. Vorrichtung nach einem der Ansprüche 1 bis 4, dadurch gekennzeichnet, daß der Flansch (1) kreisförmig ist und ei-nen Durchmesser von etwa 30 bis 35 mm hat.

6. Vorrichtung nach einem der Ansprüche 1 bis 4, dadurch gekennzeichnet, daß der Flansch (1) eine Reihe von Umfangsvorsprüngen (4) aufweist.

7. Vorrichtung nach einem der Ansprüche 1 bis 6, dadurch gekennzeichnet, daß der konvexe Boden (3) die Form eines sphärischen Gewölbes mit einem Krümmungsradius von etwa 20 bis 25 mm hat.

8. Vorrichtung nach einem der Ansprüche 1 bis 7, dadurch gekennzeichnet, daß das Plättchen aus nahrumgsmittelverträglichem Polyäthylen besteht.
